(19) Europäisches Patentamt
European Patent Office
Office européen des brevets

(11) EP 2 530 070 A1

(12) **EUROPEAN PATENT APPLICATION**
published in accordance with Art. 153(4) EPC

(43) Date of publication:
**05.12.2012 Bulletin 2012/49**

(21) Application number: **11736783.9**

(22) Date of filing: **26.01.2011**

(51) Int Cl.:
***C07C 211/61*** (2006.01) ***H01L 29/786*** (2006.01)
***H01L 51/05*** (2006.01) ***H01L 51/30*** (2006.01)
***H01L 51/42*** (2006.01)

(86) International application number:
**PCT/JP2011/000408**

(87) International publication number:
**WO 2011/093067 (04.08.2011 Gazette 2011/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.01.2010 JP 2010018906**

(71) Applicant: **Idemitsu Kosan Co., Ltd.**
**Chiyoda-ku**
**Tokyo 100-8321 (JP)**

(72) Inventors:
- **IKEDA, Hidetsugu**
**Sodegaura-shi**
**Chiba 299-0293 (JP)**
- **MATSUURA, Masahide**
**Sodegaura-shi**
**Chiba 299-0293 (JP)**

(74) Representative: **Gille Hrabal**
**Patentanwälte**
**Brucknerstrasse 20**
**40593 Düsseldorf (DE)**

(54) **DIBENZOFLUORANTHENE COMPOUND AND ORGANIC THIN-FILM SOLAR CELL USING SAME**

(57) A dibenzofluoranthene compound represented by the following formula (A). In the formula at least one of $R_1$ to $R_{14}$ is an amino group represented by the following formula (S);
$R_1$ to $R_{14}$ that is not an amino group represented by the formula (S) are independently a hydrogen atom, a $C_1$-$C_{40}$ substituted or unsubstituted alkyl group, a $C_2$-$C_{40}$ substituted or unsubstituted alkenyl group, a $C_2$-$C_{40}$ substituted or unsubstituted alkynyl group, a $C_6$-$C_{40}$ substituted or unsubstituted aryl group, a $C_3$-$C_{40}$ substituted or unsubstituted heteroaryl group, a $C_1$-$C_{40}$ substituted or unsubstituted alkoxy group or a $C_6$-$C_{40}$ substituted or unsubstituted aryloxy group. In the formula (S), $R_a$ and $R_b$ are independently a $C_6$-$C_{40}$ substituted or unsubstituted aryl group or a $C_1$-$C_{40}$ substituted or unsubstituted alkyl group; and $R_a$ and $R_b$ may combine with each other to form a ring.

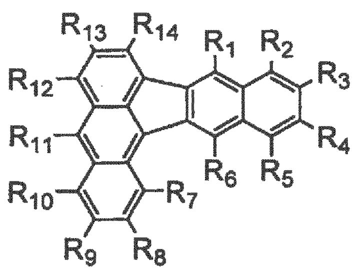
(A)

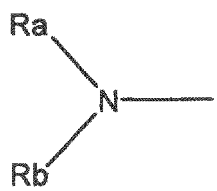
(S)

EP 2 530 070 A1

## Description

Technical Field

The invention relates to a dibenzofluoranthene compound which can be used in an organic thin film solar cell or the like and an organic thin film solar cell using the same.

Background Art

An organic thin film solar cell is, as represented by a photo diode and an imaging device which converts optical signals to electric signals and a solar cell which converts optical energy to electric energy, a device which outputs power through input of light. In this regard, it is a device which show a response opposite to an organic electroluminescence (EL) device which outputs light through input of electric power. A solar cell has attracted remarkable attention in recent years as a source of clean energy backed by the problem of running out of fossil fuels or global warming.
Heretofore, solar cells which have been put into practical use are silicon-based ones using monocrystalline Si, polycrystalline Si, amorphous Si, or the like. However, there is an increasing demand for a solar cell which uses other materials since the silicon-based solar cell is expensive and the shortage of Si as the raw material and other problems have come up to the surface. Under such circumstances, due to inexpensiveness, low toxicity and the adsence of a material shortage risk an organic solar cell has attracted attention as the next generation solar cell which takes the place of a silicon-based solar cell.
An organic solar cell is basically composed of an n layer which transfers electrons and a p layer which transfers holes, and is divided into two main types based on the materials forming each layer.
A solar cell in a layer obtained y subjecting a sensitizing dye such as a ruthenium dye to monomolecular adsorption on the surface of an inorganic semiconductor such as titania is used as the n layer, and an electrolyte solution is used as the p layer, is called as a dye-sensitized solar cell (so-called a Graetzel cell). Researches on the dye-sensitized solar cell have been energetically conducted since 1991 in view of its high photoelectric conversion efficiency (hereinafter often abbreviated as "conversion efficiency"). However, has defects that leakage occurs after the use for a long period of time due to the use of a solution, or the like.
In order to overcome such defects, researches to obtain an all solid-type dye-sensitized solar cell by solidifying an electrolyte solution have been made recently. However, the technology of allowing an organic substance to be perfused to fine pores of porous titania is very difficult, and therefore, a cell which exhibits a high conversion efficiency with a good reproducibility has not yet been completed.
On the other hand, an organic thin film solar cell in which both of the n layer and the p layer are formed from organic thin films has no defect such as leakage of the solution because it is an all solid-type cell. In addition, it can be easily fabricated, uses no ruthenium which is a rare metal, and has other advantages. Therefore, an organic thin film solar cell has attracted attention and has been energetically studied.
As for organic thin film solar cells, studies have been made in the early stage on a monolayer film formed of a merocyanine dye or the like. It was found that the conversion efficiency increases by using a multilayer film of a p layer/n layer, and thereafter, such a multilayer film has been mainly employed. The materials used at that time were copper phthalocyanine (CuPc) for the p layer and (PTCBI) for the n layer.
Thereafter, it was found that increase of the number of stacking layers by inserting an i layer (a mixture layer of a p-material and an n-material) between the p layer and the n layer results in improvement in the conversion efficiency. However, the materials used at that time were phthalocyanines and peryleneimides as ever.
Subsequently, it was found that the conversion efficiency is further improved by employing a stack cell structure in which several p/i/n layers are stacked. The materials used at that time were phthalocyanines and $C_{60}$.
In an organic thin film solar cell using a polymer compound, studies have been mainly made on the so-called bulkhetero structure in which a conductive polymer is used as the p material and a $C_{60}$ derivative is used as the n material. These materials are mixed and the resulting mixture is subjected to a heat treatment to induce the separation of micro layers, whereby the hetero interface of the p material and the n material is increased to improve the conversion efficiency. The materials used mainly in this structure were a soluble polythiophene derivative called as poly-3-hexylthiophene (P3HT) as the p material and a soluble $C_{60}$ derivative called as PCBM as the n material.
As mentioned above, in an organic thin film solar cell, the conversion efficiency was improved by optimizing the cell structure and the of the p material and the n material. However, no significant was made for the materials used in an organic thin film solar cell, and phthalocyanine-based materials, peryleneimide-based materials and $C_{60}$ derivative-based materials have still been used. Therefore, in order to improve the photoelectric conversion efficiency, development of new materials replacing these materials has been aspired.
In general, the operation process of an organic solar cell is essentially composed of (1) absorption of light and generation of excitons, (2) diffusion of excitons, (3) separation of electric charges, (4) movement of carriers and (5)

generation of an electromotive force. In general, not many organic substances show absorption properties which coincident with the solar light spectrum, and hence, an organic thin film solar cell could not attain a high conversion efficiency. For example, development of an organic EL device has been energetically conducted in recent years. Through these development efforts, an amine compound which is an excellent hole-transporting material as well as an excellent hole-injecting material has been found. Since such an amine compound has excellent hole-transporting properties, is has a possibility that it can be used as the p material for an organic thin film solar cell. However, an amine compound has a defect that, since it does not show light absorption in the visible light range, absorption characteristics for solar light spectrum is not sufficient, and hence, photoelectric conversion efficiency is not sufficient.

In general, in an organic compound, it is known that the $\pi$-electron conjugated system is expanded to allow the absorption maximum wavelength to be shifted to a long wavelength side in order to allow an organic compound to have an absorption in the visible light range. However, if the molecular weight becomes too large by expanding the conjugated system too much, problems that solubility in a solvent is lowered to lead to difficulty in purification, purification by sublimation becomes impossible due to an increase in sublimation temperature or the like have come up to the surface. Therefore, as one example of the technique to shift the absorption wavelength to a long wavelength side efficiently while suppressing the molecular weight to some degree, use of a polyacene is known.

For example, Patent Document 1 discloses a technology in which a polyacene compound is used in a solar cell material. However, in general, if the number of fused rings is increased in order to expand the visible light absorption range, a polyacene becomes instable to light or oxygen, and as a result, purification or handling becomes difficult, and the purity thereof is hard to be increased. Therefore, an organic solar cell using polyacene cannot be said as a practical solar cell material.

As examples of other compounds, for example, Patent Documents 2 discloses an organic photoelectric conversion device using a perylene derivative, for example. However, many of perylene-based compounds are hard to be dissolved in a solvent, and hence, it suffers from many difficulties when used in a method such as column chromatography or re-crystallization in which the compound is dissolved in an organic solvent, followed by purification. Further, as the purification method which does not use an organic solvent, sublimation purification or the like can be given, for example. A perylene-based compound disclosed in Patent Document 2 has a significantly high molecular planarity and has a large molecular weight, and sublimation purification has to be conducted at high temperatures, resulting in sublimation while being decomposed. Therefore, a perylene-based compound is difficult to be purified to have a high purity.

Related Art Documents

Patent Documents

Patent Document 1: JP-A-2008-091380
Patent Document 2: JP-A-2008-135540

Summary of the Invention

An object of the invention is to provide a compound which is useful as an organic electronics material, and in particular, is to provide a compound showing high efficient photoelectric conversion properties when used in an organic thin film solar cell.

According to the invention, the following dibenzofluoranthene compound or the like are provided.

1. A dibenzofluoranthene compound represented by the following formula (A):

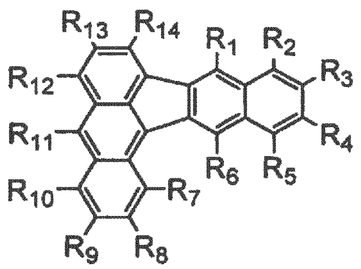

(A)

wherein at least one of $R_1$ to $R_{14}$ is an amino group represented by the following formula (S);

$R_1$ to $R_{14}$ that is not an amino group represented by the formula (S) are independently a hydrogen atom, a $C_1$-$C_{40}$ substituted or unsubstituted alkyl group, a $C_2$-$C_{40}$ substituted or unsubstituted alkenyl group, a $C_2$-$C_{40}$ substituted or unsubstituted alkynyl group, a $C_6$-$C_{40}$ substituted or unsubstituted aryl groups, a $C_3$-$C_{40}$ substituted or unsubstituted heteroaryl group, a $C_1$-$C_{40}$ substituted or unsubstituted alkoxy group or a $C_6$-$C_{40}$ substituted or unsubstituted aryloxy group;

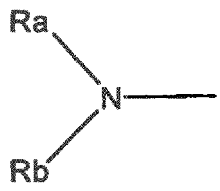

(S)

wherein $R_a$ and $R_b$ are independently a $C_6$-$C_{40}$ substituted or unsubstituted aryl group or a $C_1$-$C_{40}$ substituted or unsubstituted alkyl group; and $R_a$ and $R_b$ may combine with each other to form a ring.

2. The dibenzofluoranthene compound according to 1 which is represented by the following formula (B):

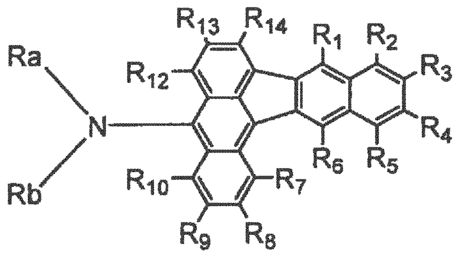

(B)

wherein $R_1$ to $R_{10}$ and $R_{12}$ to $R_{14}$ are independently a hydrogen atom, $C_1$-$C_{40}$ substituted or unsubstituted alkyl group, a $C_2$-$C_{40}$ substituted or unsubstituted alkenyl group, a $C_2$-$C_{40}$ substituted or unsubstituted alkynyl group, a $C_6$-$C_{40}$ substituted or unsubstituted aryl group, a $C_3$-$C_{40}$ substituted or unsubstituted heteroaryl group, a $C_1$-$C_{40}$ substituted or unsubstituted alkoxy group or a $C_6$-$C_{40}$ substituted or unsubstituted aryloxy and

$R_a$ and $R_b$ are a $C_6$-$C_{40}$ substituted or unsubstituted aryl group or a $C_1$-$C_{40}$ substituted or unsubstituted alkyl group; and $R_a$ and $R_b$ may combine with each other to form a ring.

3. The dibenzofluoranthene compound according to 1 or 2 which is represented by the following formula (C):

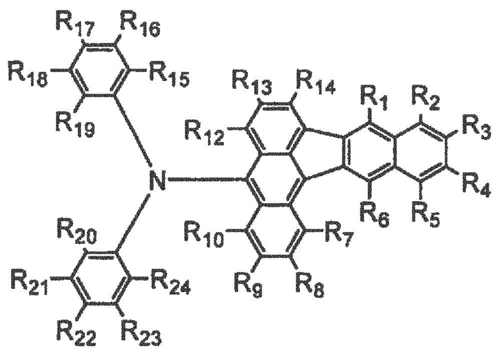

(C)

wherein R1 to $R_{10}$ and $R_{12}$ to $R_{24}$ are independently a hydrogen atom, a $C_1$-$C_{40}$ substituted or unsubstituted alkyl group, a $C_6$-$C_{40}$ substituted or unsubstituted aryl group, a $C_3$-$C_{40}$ substituted or unsubstituted heteroaryl group or a $C_1$-$C_{40}$ substituted or unsubstituted alkoxy group.

4. An organic thin film solar cell material comprising the dibenzofluororanthene compound according to any of 1 to 3.

5. An organic thin film solar cell comprising the dibenzofluororanthene compound according to any of 1 to 3.

6. The organic thin film solar cell according to 5 which comprises a p layer, and
the p layer being a layer which comprises the dibenzofluoranthene compound.

7. The organic thin film solar cell according to 6 which further comprises an n layer, and
the n layer being a layer which comprises a fullerene derivative,

The dibenzofluororanthene compound of the invention can be used as an organic electronics material. In particular, when used as a material for an organic thin film solar cell, it can provide an organic thin film solar cell having highly efficient energy conversion properties.

Mode for Carrying out the Invention

The dibenzofluoranthene compound of the invention is represented by the following formula (A):

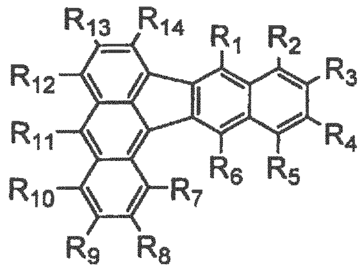

(A)

In the formula, at least one of $R_1$ to $R_{14}$ is an amino group represented by the following formula (S). $R_1$ to $R_{14}$ that is not an amino group represented by the formula (S) are independently a hydrogen atom, a $C_1$-$C_{40}$ substituted or unsubstituted alkyl group, a $C_2$-$C_{40}$ substituted or unsubstituted alkenyl group, a $C_2$-$C_{40}$ substituted or unsubstituted alkynyl group, a $C_6$-$C_{40}$ substituted or unsubstituted aryl groups, a $C_3$-$C_{40}$ substituted or unsubstituted heteroaryl group, a $C_1$-$C_{40}$ substituted or unsubstituted alkoxy group oar a $C_6$-$C_{40}$ substituted or unsubstituted aryloxy group.
The Cx to Cy means that the number of carbon atoms is x to y.

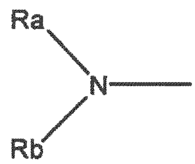

(S)

In the formula, $R_a$ and $R_b$ are independently a $C_6$-$C_{40}$ substituted or unsubstituted aryl group or a $C_1$-$C_{40}$ substituted or unsubstituted alkyl group. $R_a$ and $R_b$ may combine with each other to form a ring.

Since the compound represented by the formula (A) has not only an expanded $\pi$ electron conjugated system and an appropriate molecular weight but also contains an amino group, it is excellent in electric charge mobility. Therefore, this is useful as a material of an organic thin film solar cell. Of the compounds represented by the formula (A), the compounds represented by the following formula (B) are further preferable. That is, as for the bonding of the amino group, $R_{11}$ in the formula (A) is preferable. As a result, the compound has a molecular structure in which aside chain substituent ($R_a$ and $R_b$) projects over the upper and lower sides of the mother nucleus plane of dibenzofluoranthene efficiently. As a result, intermolecular association is suppressed by this steric hindrance, whereby a good amorphous solid can be formed. Generally, in an organic thin film solar cell, the film thickness of an organic layer is thin, and hence, generation of microcrystals due to the intermolecular association leads to the short circuit of a device, and as a result, the cell often stops operating as a solar cell. Therefore, forming a good amorphous solid is an important factor required of a material in order to produce a highly efficient organic thin film solar cell.

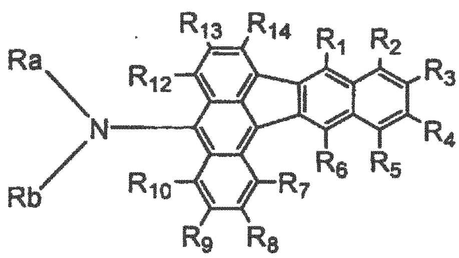

(B)

In the formula (B), $R_1$ to $R_{10}$, $R_{12}$ to $R_{14}$ and $R_a$ and $R_b$ are the same groups as those in the formula (1). Of the compounds represented by the formula (B), a compound represented by the following formula (C) is preferable. Thus all of substituents bonded to the amino group become aryl groups, and stability of the compound is improved.

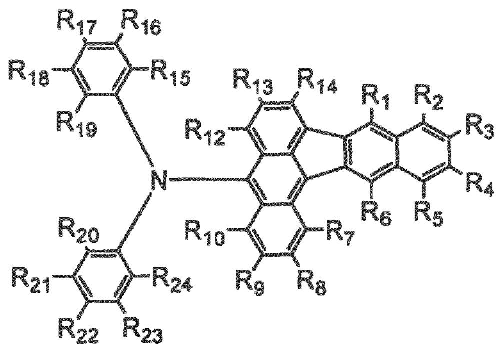

(C)

In the formula, $R_1$ to $R_{10}$ and $R_{12}$ to $R_{24}$ are independently a hydrogen atom, a $C_1$-$C_{40}$ substituted or unsubstituted alkyl group, a $C_6$-$C_{40}$ substituted or unsubstituted aryl group, a $C_3$-$C_{40}$ substituted or unsubstituted heteroaryl group or a $C_1$-$C_{40}$ substituted or unsubstituted alkoxy group. These groups are preferable in respect of easiness in production of an organic thin film solar cell by the deposition method. Moreover, in this respect, it is suitable as an organic electronics material.

In the specification of this application, as the substituent in the "substituted or unsubstituted" includes a halogen atom, an alkyl group, an aryl group, a heteroaryl group, an alkoxy group, an aryloxy group or the like, as described later, can be given, The "unsubstituted" means the group or atom is substituted by a hydrogen atom.

Specific examples of each group ($R_1$ to $R_{24}$, $R_a$ and $R_b$) in the formulas (A) to (C) and substituents are given below.

The $C_1$-$C_{40}$ substituted or unsubstituted alkyl group may be linear, branched or cyclic. Specific examples thereof include a methyl group, an ethyl group, a 1- propyl group, a 2-propyl group, a 1-butyl group, a 2-butyl group, a sec-butyl group, a tert-butyl group, a pentyl group, a hexyl group, an octyl group, a decyl group, a dodecyl group, a 2-ethylhexyl group, a 3,7-dimethyloctyl group, a cyclopropyl group, a cyclopentyl group, a cyclohexyl group, a 1-adamantyl group, a 2-adamantyl group, a norbonyl groups, a trifluoromethyl group, a trichloromethyl group, a benzyl group, an $\alpha,\alpha$-dimethylbenzyl groups, a 2-phenylethyl group and a 1-phenylethyl group. Of these, a methyl group, an ethyl group, a propyl group, an isopropyl group, a tert-butyl group and a cyclohexy group are preferable in respect of easiness in obtaining raw materials, or for other reasons.

As the substituent for the above-mentioned alkyl group, a halogen atom such as a fluorine atom, a chlorine atom and an iodine atom; a $C_1$-$C_{10}$ (more preferably $C_1$-$C_5$) alkoxy group such as a methoxy group, an ethoxy group and a tert-butyloxy group; and an aryl group (preferably $C_6$-$C_{14}$) such as a phenyl group, a tolyl group and a naphthyl group can be given.

The $C_2$-$C_{40}$ substituted or unsubstituted alkenyl group may be linear, branched or cyclic. Specific examples thereof include a vinyl group, a propenyl group, a butenyl group, an oleyl group, an eicosapentaenyl group, a docosahexaenyl group, a styryl group, a 2,2-diphenylvinyl groups, a 1,2,2-triphenylvinyl group and a 2-phenyl-2-propenyl group. Of these, a vinyl group, a styryl group and a 2,2-diphenylvinyl group are preferable in respect of easiness in obtaining raw materials, or for other reasons.

As the substituent for the above-mentioned alkenyl group, groups exemplified above referring to the $C_1$-$C_{40}$ substituted or unsubstituted alkyl group can be given.

The $C_2$-$C_{40}$ substituted or unsubstituted alkynyl group may be linear, branched or cyclic. Specific examples thereof include an ethenyl group, a propynyl and a 2-phenylethenyl group. Of these, an ethenyl group, a 2-phenylethenyl group or the like are preferable in respect of easiness in obtaining raw materials, or for other reasons.

As the substituent for the above-mentioned alkynyl group, groups exemplified above referring to the $C_1$-$C_{40}$ substituted or unsubstituted alkyl group can be given.

Specific examples of the $C_6$-$C_{40}$ substituted or unsubstituted aryl group include a phenyl groups, a 2-tolyl group, a 4-tolyl group, a 4-trifluoromethylphenyl group, a 4-methoxyphenyl group, a 4-cyanophenyl group, a 2-biphenylyl group, a 3-biphenylyl group, a 4-biphenylyl group, a terphenylyl groups, a 3,5-diphenylphenyl group, a 3,4-diphenylphenyl group, a pentaphenyphenyl group, a 4-(2,2-diphenylvinyl)phenyl group, a 4-(1,2,2-triphenylvinyl)phenyl group, a fluorenyl group, a 1-naphthyl group, a 2-naphthyl group, a 9-anthryl group, a 2-anthryl group, a 9-phenanthryl group, a 1-pyrenyl group, a chrycenyl group, a naphthacenyl group, and a coronenyl group. Of these, a phenyl group, a 4-biphenyl group, a 1-naphthyl group, a 2-naphthyl groups, a 9-phenanthryl group or the like are preferable in respect of easiness in

obtaining raw materials, or for other reasons.

As the substituent for the above-mentioned aryl group, groups exemplified above referring to the $C_1$-$C_{40}$ substituted or unsubstituted alkyl group or a cyano group can be given.

As for the $C_3$-$C_{40}$ substituted or unsubstituted heteroaryl group, in the case of a nitrogen-containing azole-based heterocyclic ring, the heterocyclic ring can be bonded through not only carbon but also nitrogen. Specific examples thereof include groups based on a skeleton such as furan, thiophene, pyrrole, imidazole, benzimidazole, pyrazole, benzpyrazole, triazole, oxadiazole, pyridine, pyrazine, triazine, quinoline, benzofuran, dibenzofuran, benzothiophene, dibenzothiophene and carbazole. Of these, furan, thiophene, pyridine, carbazole or the like are preferable in respect of easiness in obtaining raw materials, or for other reasons.

As the substituent for the above-mentioned heteroaryl group, groups exemplified above referring to the $C_1$-$C_{40}$ substituted or unsubstituted alkyl group or a cyano group can be given.

The $C_1$-$C_{40}$ substituted or unsubstituted alkoxy group may be linear, branched or cyclic. Specific examples thereof include a methoxy group, an ethoxy group, a 1-propyloxy group, a 2-propyloxy groups, a 1-butyloxy group, a 2-butyloxy group, a sec-butyloxy group, a tert-butyloxy group, a pentyloxy group, a hexyloxy group, an octyloxy group, a decyloxy group, a dodecyloxy group, a 2-ethylhexyloxy groups, a 3,7-dimethyloctyloxy group, a cyclopropyloxy group, a cyclopentyloxy group, a cyclohexyloxy group, a 1-adamantyloxy group, a 2-adamantyloxy group, a norbonyloxy group, a trifluoromethoxy groups, a benzyloxy group, an $\alpha$,$\alpha$-dimethylbenzyloxy group, a 2-phenylethoxy group and a 1-phenylethoxy group. Of these, a methoxy group, an ethoxy group, a tert-butyloxy group and the like are preferable in respect of easiness in obtaining raw materials, or for other reasons.

As the substituent for the above-mentioned alkoxy group, a halogen atom such as a fluorite atom, a chlorine atom and an iodine atom; and an aryl group (preferably $C_6$-$C_{14}$) such as a phenyl groups, a tolyl group and a naphthyl group can be given.

The $C_6$-$C_{40}$ substituted or unsubstituted aryloxy group may be linear, branched or cyclic. Specific examples thereof include a substituent in which the above-mentioned aryl groups are through oxygen. Of these, a phenoxy group, a naphthoxy group, a phenanthryloxy group or the like are preferable in respect of in obtaining raw materials, or for other reasons.

At the substituent for the above-mentioned aryloxy group, groups exemplified above referring to the $C_1$-$C_{40}$ substituted or unsubstituted alkoxy can be given.

In the formula (A), it is preferred that of all of all of $R_1$ to $R_{14}$ that is not an amino group, be a hydrogen atom. In the formulas (B) and (C), It is preferred that all of $R_1$ to $R_{10}$ and all of $R_{12}$ to $R_{14}$ be a hydrogen atom.

In $R_a$ and $R_b$ in the amino group represented by the above-mentioned formula (S), as examples of the $C_6$-$C_{40}$ substituted or unsubstituted aryl group or the $C_1$-$C_{40}$ substituted or unsubstituted alkyl group, the same groups as those for $R_1$ to $R_{14}$ in the formula (A) mentioned above can be given. $R_a$ and $R_b$ may combine with each other to form a ring. In order to the stability of the compound, it is preferred that $R_a$ and $R_b$ be independently a $C_6$-$C_{40}$ substituted or unsubstituted aryl group. Further, in respect of a compound having an appropriate molecular weight, as the aryl group, a phenyl group or a naphthyl group is preferable. As the substituent of the aryl group, a $C_1$-$C_6$ alkyl group or a $C_1$-$C_6$ alkoxy group is preferable.

Specific examples of the amino group represented by the formula (S) include a methylphenylamino group, a diphenylamino group, a di-p-tolylamino group, a di-m-tolylamino group, a phenyl-m-tolylamino group, a phenyl-1-naphthylamino group, a phenyl-2-naphthylamino groups, a phenyl(sec-butylphenyl)amino group, a phenyl-t-butylamino group, a bis(4-methoxyphenyl)amino group, a dimethylamino group, a methylethylamino group, a diethylamino group, a bis(2-hydroxyethyl)amino group, a bis(2-methoxy ethyl)amino group, a piperidino and a morpholino group.

Of these, a diphenylamino group, a ditolylamino group, bis(4-methoxyphenyl)amino groups, a dimethylamino group, a diethylamino group, a piperidino group or the like are preferable in respect of easiness in obtaining raw materials, or for other reasons.

Specific examples of the dibenzofluoranthene of the invention include the following compounds, for example.

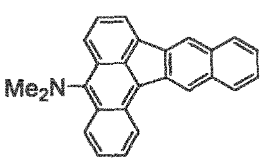 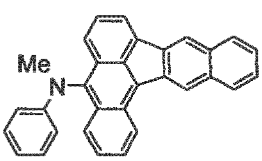 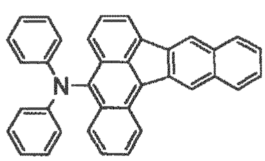 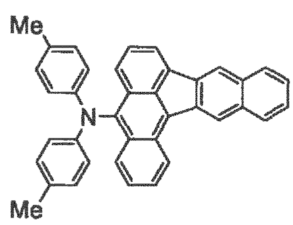

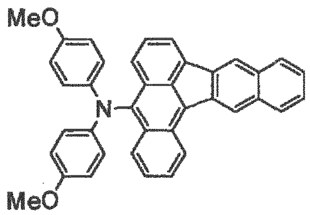
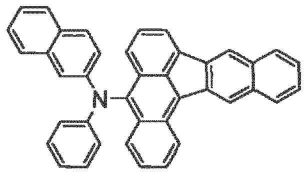
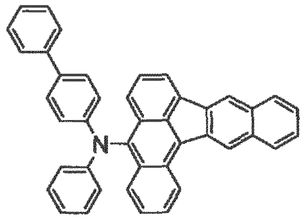

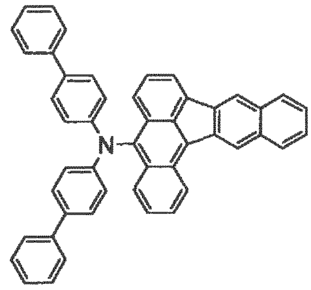
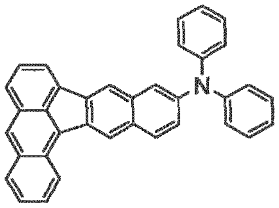
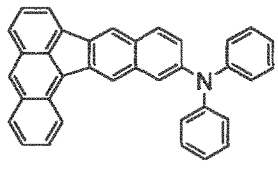
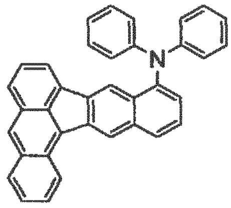

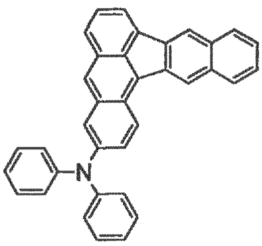
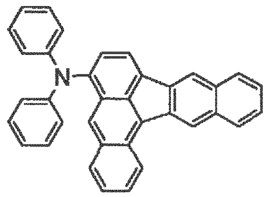
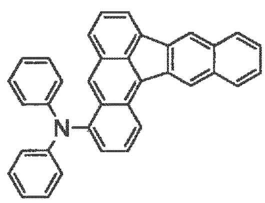
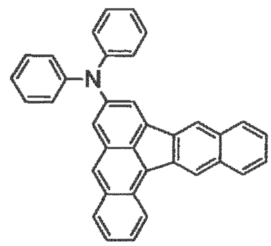

wherein Me is a methyl group.

There are various methods for synthesizing the compound of the invention. Of these methods, a synthesis route in which a naphthyl anthracene derivative is subjected to a ring closure reaction is preferable in respect of easiness in obtaining raw materials, moderate reaction conditions, and obtaining an intended product in a high yield, or for other reasons. One of the synthesis route is shown below.

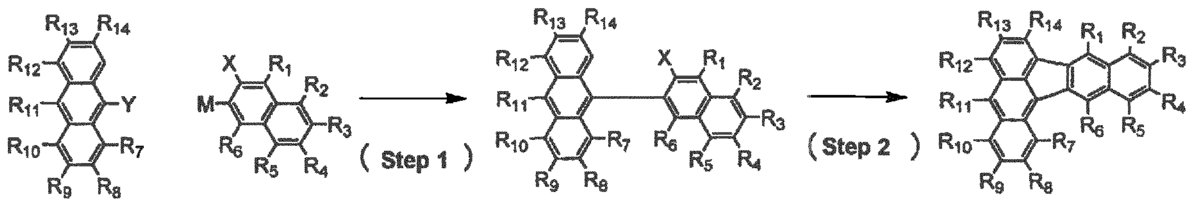


X and Y represent a halogen such as iodine, bromine and chlorine or a pseudohalogen group such as trifluoromethane sulfonyloxy(trifuryloxy) group, nonafluorobutanesulfonyloxy(nonafuryloxy) group, a toluene sulfonyloxy (tosyloxy) and a methanesulfonyloxy(mesyloxy) group. Of these, a halogen such as chlorine and bromine is preferable in respect of availability of raw materials and high reactivity or the like. M is a Grignard reagent such as magnesium bromide and magnesium chloride, an organic zinc reagent such as zinc chloride and zinc bromide, an organic tin reagent such as a tributylstannyl group and a trimethylstannyl group and a boronic acid such as a dihydroxyboryl group and a pinacolatoboryl group. As for the conditions under which these typical organic metal reagents are reacted width anthracene derivative, a Kumada-Tamao coupling reaction is preferable in the case of a Grignard reagent, a Negishi coupling reaction is preferable in the case of an organic zinc reagent, a Kosugi-Uda-Stille coupling reaction is preferable in the case of an organic tin reagent, and a Suzuki-Miyaura coupling reaction is preferable in the case of a boronic acid reagent. That is, it is preferable to use a reaction named after well known chemists. Of a preferable reaction is a Suzuki-Miyaura coupling reaction in which a boronic acid reagent is used since reaction conditions are moderate and a high yield can be attained.

As the used in the ring closure reaction in the step 2, combination of various metals and ligands can be used. Of was for the metal, nickel or palladium is preferable since it attains a high yield. For example, nickel chloride, dichlorobis(triphenylphosphine)nickel, tetrakis (triphenylphosphine)nickel, bis(cyclooctadiene)nickel, nickel tetracarbonyl, bis (acetylacetonato)nickel, nickelocene, tetrakis(triphenylphosphine)palladium, dichlorobis(triphenylphosphine)palladium, tris(dibenzylideneacetone)dipalladium, palladium acetate, palladium chloride, or the like can be given. As for the ligand, phosphines such as triphenylphosphine, tri-o-tolylphosphine, 2,2'-bis(diphenylphosphino)-1,1'-binaphthyl (BINAP), 4,5-bis(diphenylphosphino)-9,9-dimethylxanthene (XantPhos), 1,2-bis(diphenylphosphino)benzene, 1,1'-bis(diphenylphosphino)ferrocene (DPPF), tri-t-butylphosphine, 2-(dicyclohexylphosphino)biphenyl (JohnPhos), 2-(dicydohexylphosphino)-2'-(N,N-dimethylamino)biphenyl (DavePhos), 2-(2-dicyclohexylphosphino)-2',6'-dimethoxybiphenyl (S-Phos) and 2-(2-dicydohexylphosphino)-2',4',6'-triisopropylbiphenyl (X-Phos) are preferable.

During the reaction, it is preferable to add a base. Examples of the base usable at this time include carbonates such as potassium carbonate and carbonate, alkoxides such as sodium t-butoxide and organic bases such as diazabicycloundecene, diazabicyclononen, triethylamine, diisopropylethylamine (Hunig's base), pyridine and 4-dimethylamino pyridine (DMAP). In respect of attaining a high yield, organic bases such as diazabicycloundecence are preferable.

In order to increase the reactivity of the base, it is possible to a phase transfer catalyst. As examples of the phase transfer catalyst, tetrabutylammonium hydrogensulfate, benzyltriethylammonium chloride or the like can be given,

The dibenzofluororanthene compound of the invention can be used in an organic electronics material and an organic semiconductor material. For it can be used in an organic electroluminescence an organic field effect transistor an organic solar cell material or the like. In particular, when used as a for an organic thin film solar cell material, it shows highly efficient energy conversion characteristics.

The organic thin film solar cell material comprising the compound of the invention may comprise the compound of the invention singly or in combination with other ingredients.

No particular restrictions are imposed on the cell structure of the organic thin film solar cell of the invention as long as it is a structure in the compound of the invention is contained between a pair of electrodes. Specifically, structures in which the following constitutional elements are on a stable insulating can be given.

(1) Lower electrode/Organic compound layer/Upper electrode
(2) Lower electrode/p layer/n layer/Upper electrode
(3) Lower electrode/p layer/i layer (or a mixture layer of a p material and an n material)/n layer/Upper electrode
(4) Lower electrode/a mixture layer of a p material and an n material/Upper electrode

In addition, a structure in which the p layer and the n layer in the structures (2) and (3) above are replaced can be given.

Moreover, a buffer layer may be provided between an electrode and an organic layer, if necessary. For example, as specific examples, when a buffer layer is provided in the above-mentioned structure (2), a structure having the following constitutional elements can be given.

(5) Lower electrode/Buffer layer/ p layer/n layer/Upper electrode
(6) Lower electrode/p layer/n layer/Buffer layer/Upper electrode
(7) Lower electrode/Buffer layer/p layer/n layer/Buffer layer/Upper electrode

The compound of the invention can be used in an organic compound layer, a p layer, an n layer, an i layer, a mixture layer of a p material and an n material and a buffer layer, for example, In particular, it is preferred that the compound of the invention be used as the material of the p layer.

In the organic thin film solar cell of the invention, the compound of the invention may be contained in any of the elements constituting the cell. Further, the components containing the compound of the invention may contain other ingredients in combination. As for the constitutional elements which do not contain the compound of the invention or the mixture material, it is possible to use a known element or a material which is used in an organic thin film solar cell. Each constitutional element will be briefly explained below.

1. Lower electrode and Upper electrode

No particular restrictions are imposed on the material for the lower electrode and the upper electrode, and a known conductive material can be used. For example, as the electrode connecting with the p layer, a metal such as tin-doped indium oxide (ITO), gold (Au) and palladium (Pd) can be used. As the electrode connecting with the n layer, metals such as silver (Ag), aluminum (Al), indium (In), calcium (Ca), platinum (Pt) and lithium (Li); a binary metal system such as Mg:Ag, Mg:In or Al:Li, and the above-mentioned materials for the electrode connecting with the p layer can be used.

To obtain high efficient photoelectric conversion properties, in the case when the organic thin film solar cell is a solar cell using solar light as the energy source, it is desired that at least one surface of the solar cell preferably have sufficient transparency in the solar light spectrum, The transparent electrode can be formed of a known conductive material by

deposition, sputtering or the like such that the predetermined light transmittance is secured. It is preferred the light transmittance of the electrode on the light-receiving surface be 10% or more. In a preferable configuration in which a pair of electrodes is provided, one of the electrodes contains a metal or a metal compound having a large work function and the other contains a metal or at compound having a small work function.

2. Organic compound layer

The organic compound layer is a p layer, mixture layer of a p material and an n material or an n layer. When the compound of the invention is used in the organic compound layer, specifically, a configuration having the lower electrode and the compound of the and a mixture layer of the n layer material or the p layer material, mentioned later and the upper electrode, or the like can be given.

3. p layer, n layer and i layer

When the compound of the invention is used in the p layer, no specific restrictions are imposed on the material of the n layer. However, it is preferable to use a material having a function as an electron acceptor. For example, as organic compounds, fullerene derivatives such as $C_{60}$, $C_{70}$, PCBM and $PC_{70}BM$, carbon nanotube, perylene derivatives, polycyclic quinones and quinacridone can be given, and as polymers, CN-poly(phenylene-vinylene), MEH-CN-PPV, polymers having a -CN group or a $CF_3$ group, those polymers substituted by a $-CF_3$ group and poly(fiuorene)derivatives can be given. Preferred are materials having high electron mobility. More preferred are materials having a small electron affinity. Use of such a material having a small electron in for the n layer can lead to a sufficient open-circuit voltage.

As inorganic compounds, an inorganic semiconductor compound having n-type characteristics can be given. Specific examples include doped semiconductors and compound semiconductors such as n-Si, GaAs, CdS, PbS, CdSe, InP, $Nb_2O_5$ and $Fe_2O_3$; and titanium oxides such as titanium dioxide ($TiO_2$), titanium monoxide (TiO) and dititanium trioxide ($Ti_2O_3$); and conductive oxides such as zinc oxide (ZnO) and tin oxide ($SnO_2$). The above-mentioned inorganic semiconductor compounds having an n-type characteristic may be used alone or in combination of two or more. Titanium oxide is preferably used, with titanium dioxide being particularly preferable.

When the compound of the invention is used in the p layer, it is preferable to use the above-mentioned fullerene derivatives in the n layer. Use of the fullerene derivatives in the n layer, it is to fabricate a highly efficient organic thin film solar cell.

When the compound of the invention is used in the n layer, no specific restrictions are imposed on the material of the p layer. However, it is preferable to use a material having a function as a hole acceptor. For example, as organic compounds, amine compounds represented by N,N'-bis(3-tolyl)-N,N'-diphenylbenzidine (mTPD), N,N'-dinaphthyl-N,N'-diphenylbenzidine (NPD) and 4,4',4"-tris(phenyl-3-tolylamino)triphenylamine (MTDATA); phthalocyanines such as phthalocyanine (Pc), copper phthalocyanine (CuPc), zinc phthalocyanine (ZnPc) and titanylphthalocyanine (TiOPc); and porphyrins represented by octaethylporphyrin (OEP), platinum octaethylporphyrin (PtOEP) and zinc tetraphenylporphyrin (ZnTPP) can be given. As polymer compounds, main chain-type conjugated polymers such as polyhexylthiophene (P3HT) and methoxyethylhexyloxyphenylenevinylene (MEHPPV), and side chain-type polymers represented by polyvinyl carbazole can be given.

When the compound of the invention is used in the i layer, the i layer may be formed by mixing the p layer compound and the n layer compound as mentioned above. However, it is possible to use the compound of the invention singly as the i layer. As the p layer and the n layer in such a case, any of the compounds exemplified above can be used.

4. Buffer layer

In general, an organic thin film solar cell has a small total film thickness, and hence, the upper electrode and the lower electrode often short-circuit so that a yield in fabrication of the cells may decrease. Such short-circuit can be be avoided by stacking of a buffer layer.

As materials for forming the buffer layer, preferred are compounds having a sufficiently high carrier mobility such that the short-circuit current does not decrease even when the film thickness of the buffer layer increases. Examples of the material for the buffer layer include, as a low molecular compound, aromatic cyclic acid anhydrides represented by NTCDA as shown below, and as a polymer compound, known conductive polymers represented by poly(3,4-ethylenedioxy)thiophene: polystyrene sulfonate (PEDOT:PSS) and polyaniline:camphor sulfonic acid (PANI:CSA),

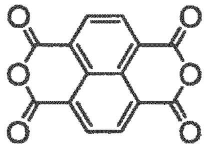 NTCDA 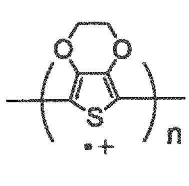 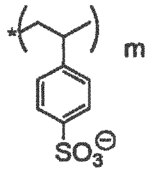 PEDOT : PSS

The buffer layer may have a role of preventing excitons from deactivation due to diffusion to the electrode. It is effective for enhancing the efficiency that the buffer layer is inserted as an exciton blocking layer. The exciton blocking layer may be inserted into each of the anode side and the cathode side, and also be inserted into both the sides at the same time. In this case, preferred materials for the exciton blocking layer include known materials for the hole barrier layer and for the electron barrier layer in organic EL devices. Preferred materials for the hole barrier layer are compounds having a sufficiently large ionization potential. Preferred materials for the electron barrier layer are compounds having a sufficiently small electron affinity. Specifically, bathocuproin (BCP), bathophenanthroline (BPhen) and the like, which are known as the materials for organic EL devices, can be as the material for the hole barrier layer on the cathode side.

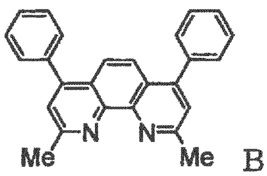 BCP 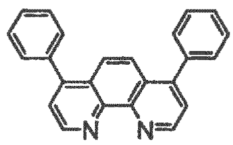 BPhen

In addition to the above-mentioned compounds, the inorganic semiconductor compounds exemplified as the materials for the n layer as mentioned above may be used as the materials for the buffer layer. Further, CdTe, p-Si, SiC, $WO_3$ and the like which are p-type inorganic semiconductor compounds can be used.

5. Substrate

As the material for the substrate, materials having mechanical strength, thermal strength and transparency are preferable. Examples of the substrate include glass substrates and transparent resin films. The transparent resin films include films made of polyethylene, ethylene-vinyl acetate copolymer, ethylene-vinylalcohol copolymer, polypropylene, polystyrene, poly(methyl methacrylate), polyvinylchloride, polyvinylalcohol, polyvinyl butyral, nylon, polyether ether ketone, polysulfone, polyether sulfone, tetrafluoroethylene-perfluoroalkylvinyl ether copolymer, polyvinylfluoride, tetrafluoroethylene-ethylene copolymer, tetrafluoroethylene-hexafluoropropylene copolymer, polychlorotrifluoroethylene, polyvinylidene fluoride, polyester, polycarbonate, polyurethane, polyimide, polyetherimide, polyimide and polypropylene.

Each layer of the organic thin film solar cell of the invention can be formed by dry film-forming methods such as vacuum vapor deposition, sputtering, plasma coating, and ion plating, and wet film-forming methods such as spin coating, dip coating, casting, roll coating, flow coating and inkjet.

The film thickness of each layer is not particularly limited, but the film can be made into an appropriate film thickness. It is generally known that the exciton diffusion length of an organic thin film is short. If the film thickness is too thick, excitons may be deactivated before they reach the hetero interface, so that photoelectric conversion efficiency becomes low. On the other hand, if the film thickness is too thin, generation of pinholes or other problems may occur. As a result, the sufficient diode properties cannot be obtained, resulting in lowering of the conversion efficiency. The appropriate film thickness of each layer is usually in a range of 1 nm to 10 $\mu$m, and further preferably in a range of 5 nm to 0.2 $\mu$m.

In the case of employing the dry film-forming method, known resistance heating methods are preferable. In the of forming a mixture layer, a film-forming method in which co-deposition is conducted using plural evaporation sources is preferable, for example. Further preferred is to control the substrate temperature during film-formation.

In the of employing the wet film-forming method, a material for forming each layer is dissolved or dispersed in an appropriate solvent to prepare a luminescent organic solution, and a thin film is formed from the solution. The solvent can be arbitrarily selected. The usable solvent includes halogenated hydrocarbon-based solvents such as dichloromethane, dichloroethane, chloroform, carbon tetrachloride, tetrachloroethane, trichloroethane, chlorobenzene, dichlorobenzene and chlorotoluene; ether solvents such as dibutyl ether, tetrahydrofuran, dioxane and anisole; alcohol solvents such as methanol, ethanol, propanol, butanol, pentanol, hexanol, cyclohexanol, methyl cellosolve, ethyl cellosolve and ethylene glycol; hydrocarbon solvents such as benzene, toluene, xylene, ethylbenzene, hexane, octane, decane and tetralin; and ester solvents such as ethyl acetate, butyl acetate and amyl acetate. Of these, the hydrocarbon-based solvents and the ether solvents are preferable. These solvents may be used alone or in a mixture of two or more. Usable

solvents are not limited thereto.

In the invention, resins or additives may be used in any of the organic layers in the organic thin film solar cell in order to improve film-forming properties or for the prevention of generating pinholes of the film, or the like. Usable resins include insulating resins such as polystyrene, polycarbonate, polyarylate, polyester, polyamide, polyurethane, polysulfone, poly(methyl methacrylate), poly(methyl acrylate) and cellulose, and copolymers thereof; photo-conductive resins such as poly-N-vinylcarbazole and polysilane; and conductive resins such as polythiophene and polypyrrole. The additives include an antioxidant, an ultraviolet absorbent and a plasticizer.

EXAMPLES

[Dibenzofluororanthene compound]

Example 1

Compound A was synthesized according to the following reaction:

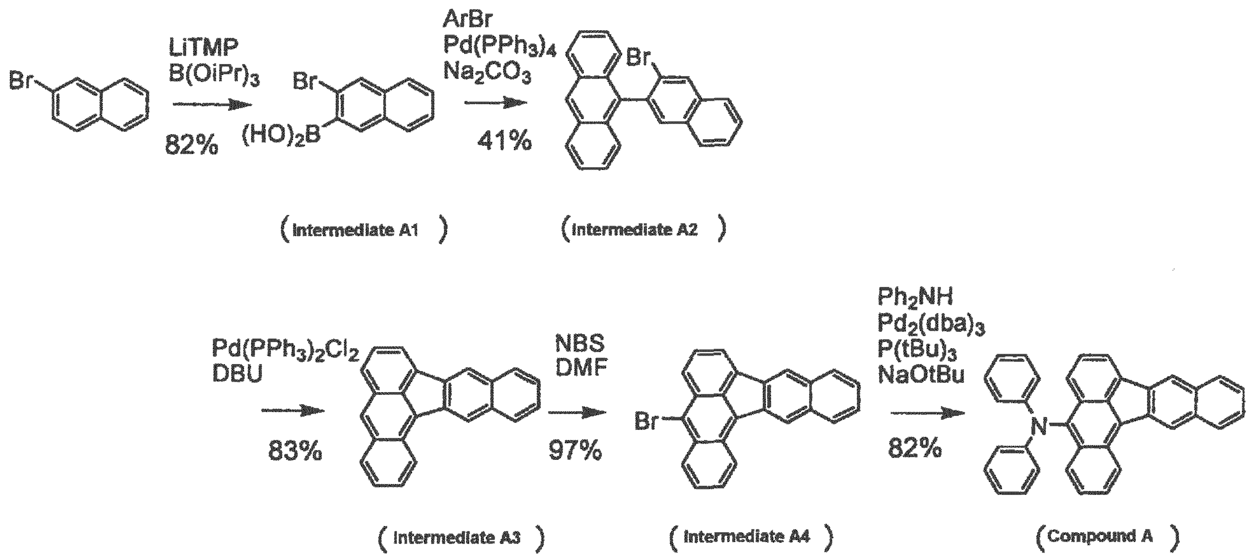


(Intermediate A1) (Intermediate A2)

(Intermediate A3) (Intermediate A4) (Compound A)

(1) Synthesis of intermediate A1

In the atmosphere of nitrogen, 2,2,6,6-tetramethylpiperidine (8.2g, 58 mmol 1.5 eq.) was dissolved in 80 ml of tetrahydrofuran anhydride (THF). The resulting mixture was cooled to -30°C in dry ice/methanol bath. To the resultant, an n-butyllithium/hexane solution (1.6 mol/1.37ml, 59 mmol,1 eq. to TMP) was added dropwise, and the resulting mixture was stirred at -20°C for 20 hours. The reaction mixture was cooled to -74°C. Then, triisopropyl borate (18 ml, 78 mmol 2eq.) was added. After the lapse of 5 minutes, a 2-bromonaphthalene/anhydrous THF solution (8g, 39 mmol/15ml) was added dropwise over 10 minutes. The reaction mixture was stirred at from -74°C to room temperature for 10 hours, and allowed to stand overnight. The reaction mixture was cooled in water bath. A 5% aqueous hydrochloric acid solution (100 ml) was gradually added to the mixture to allow it to be deactivated, and then diluted with toluene (100 ml). The thus diluted mixture was extracted with an aqueous 5% sodium hydroxide solution (150 ml). An aqueous phase was cooled in ice bath. Concentrated hydrochloric acid was gradually added to adjust the pH to 2. The resulting solids were filtered out, washed with water, whereby a white solid (8.0g, 82%) was obtained.
The results of the nuclear magnetic resonance imaging measurement ($^1$H-NMR) of this solid are shown below:

$^1$H-NMR(400 MHz,$CDCl_3$,TMS) $\delta$ :5.50(2H,s), 7.52(1 H,t,J=7Hz), 7.56(1 H,t,J=7Hz), 7.75(1 H,d,J=7Hz), 7.88(1H, d,J=7Hz), 8.04(1H,s), 8.49(1H,s)

(2) Synthesis of intermediate A2

In the atmosphere of nitrogen, the intermediate A1 (8.0g, 32 mmol), 9-bromoanthracene (9.0g, 35 mmol), tetrakis(triphenylphosphine)palladium (0) (0.74g, 0.64 mmol, 2%Pd) were dissolved in 1,2-dimethoxyethane(100ml). Then, a 2M aqueous sodium carbonate solution (10.2g, 96 mmol, 3 eq./50 ml) was added, followed by reflux for 11

hours. The reaction was diluted with toluene (150 ml) and water (50 ml), and an organic phase was separated, washed with saturated saline (50 ml), followed by drying with anhydrous magnesium sulfate. The solvent was distilled off to obtain yellowish oil. The oil was purified by column chromatography (silica gel/hexane + 5% dichloromethane, subsequently hexane + 10% dichloromethane), whereby a pale yellowish solid (5.0g, 41%) was obtained.

$^1$H-NMR(400 MHz, $CDCl_3$, TMS) δ: 7.32-7.36(2H,m), 7.46(2H,d,J=8Hz), 7.49(2H, d, J=9Hz), 7.54-7.62(2H,m), 7. 2(1 H,d,J=8Hz), 7. (1 H,s), 7.92(1 H,d,J=8Hz), 8.07(2H,d,J=8Hz), 8.36(1H,s), 8.57(1 H,s)

(3) Synthesis of intermediate A3

In the atmosphere of nitrogen, the intermediate A2 (6.2g,16 mmol), dicydobis(triphenylphosphine)palladium (11) (1.1g,1.6 mmol, 10%Pd), 1,8-diazabicyclo[5.4.0]-7-undecene (3.6g, 24 mmol, 1.5 eq.) were dissolved in 70 ml of anhydrous DMF (N,N-dimethylformamide), followed by stirring at 140°C. The reaction mixture was diluted with toluene (100 ml), filtered out through celite 545, and washed with toluene (100 ml). The solvent was distilled off from the filtrate, whereby a dark yellowish solid was obtained. The solid was purified by column chromatography (silica gel/dichloromethane), whereby a yellowish solid (4.0g, 83%) was obtained.

$^1$H-NMR(400 MHz,$CDCl_3$,TMS) δ: 7.50-7.57(3H,m), 7.65-7.74(2H,m), 7.94-8.05(4H,m), 8.15(1 H,d,J=9Hz), 8.37(1H,s), 8.44(1H,s), 8.73(1H,s), 8.89(1H,d,J=9Hz)

(4) Synthesis of intermediate A4

The intermediate A3 (4.0g, 13 mmol) was suspended in anhydrous DMF (100 ml), and an anhydrous DMF solution (5 ml) of N-bromosuccinimide (2.6g, 15 mmol, 1.1 eq.) was added, followed by stirring at room temperature for 4 hours. The resultant was allowed to stand overnight. The reaction mixture was cooled in a water bath, and water (100 ml) was added. The generated solid was filtered out, washed with water and methanol, whereby an orange solid (4.8g, 97%) was obtained,

$^1$H-NMR(400 MHz, $CDCl_3$,TMS): δ 7.52-7.75(5H,m), 7.93-8.01(3H,m), 8.27(3H,m), 8.34(1H,s), 8.66(1H,d, J=7Hz), 8.70(1H,s), 8.90(1H,d,J=8Hz)

(5) Synthesis of compounds A

In the atmosphere of nitrogen, the intermediate A4 (2.8g, 7.3 mmol), diphenylamine (1.5g, 8.9 mmol, 1 .2 eq.), tris(dibenzylideneacetone)dipalladium (0) (0.10g, 0.11 mmol, 3%Pd) and sodium-t-butoxide (1.1g,11 mmol, 1.5 eq.) were suspended in anhydrous toluene (90 ml). Then, a tri-t-butylphosphine/toluene solution (66 wt%, 0.05 ml, 0.16 mmol, 0.7 eq. to Pd) was added, followed by reflux for 11 hours. The reaction mixture was filtered out through a silica gel pad, and washed with toluene(150ml). The solvent was distilled off from the filtrate, and the resulting brown solid was washed with methanol. Then, the solid was purified by column chromatography (silica gel/hexane + 33% dichloromethane, subsequently hexane + 50% dichloromethane, and finally dichloromethane alone), whereby an orange solid (2.8g, 82%) was obtained. This solid was re-crystallized from toluene (80), whereby 2.0g of an orange needle-like crystal was obtained.

The results of measuring the purity of this solid by $^1$H-NMR, field desorption mass spectroscopy (FDMS) and high performance liquid chromatography (HPLC) are shown below.

$^1$H-NMR(400 MHz, $CDCl_3$, TMS) δ: 6.90(2H,t,J=7Hz), 7.13(4H,d,J=7Hz), 7.18(4H,t,J=7Hz), 7,43-7.55(4H,m), 7.66-7.71 (1H,m), 7.94(1H,d,J=8Hz), 7.95(1 H,d,J=9Hz), 7.99(1 H,d,J=6Hz), 8.03(1H,d,J=9Hz), 8.31 (1H,d,J=9Hz), 8.38(1H,s), 8.77(1 H,s), 8.97(1H,d,J=9Hz)

- FDMS: calculated value $C_{36}H_{23}N$=469, measured value m/z=469 ($M^+$, 100)
- HPLC: 98.9% (detected wavelength 254 nm: area%)

The physical properties are as follows,

- Melting point: 314°C(DSC)
- Glass transition temperature: 125°C(DSC)
  (The melting point was confirmed by elevating the temperature from normal temperature at a rate of 10°C per minute, and then the temperature was lowered at a rate of 10°C per minute to obtain a glass transition temperature)
- Absorption maximum wavelength: 498 nm (dichloromethane)
- Fluorescence maximum wavelength: 587 nm (dichloromethane)
  (the solution was prepared using dichloromethane as a solvent, and the concentration was adjusted to $10^{-5}$mol/L. An absorption maximum wavelength and a fluorescence maximum wavelength were measured by means of an absorption spectrophotometer).

The resulting solid (2.0g) was purified by sublimation under conditions of 320°C16.2 × $10^{-3}$ Pa, whereby an orange solid (1.9g) was obtained.

- HPLC: 99. 1% (detection wavelength 254 nm: area %)

Example 2

Compound B was synthesized by the following reaction:

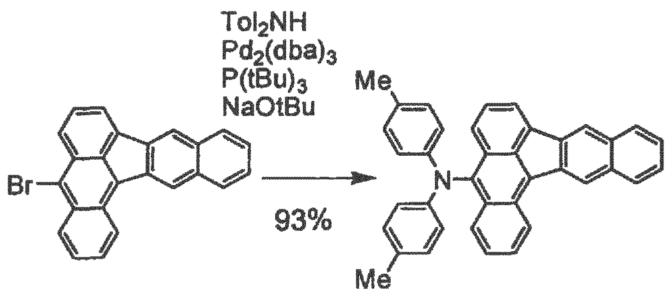


(Intermediate A4) (Compound B)

In the atmosphere of nitrogen, the intermediate A4 (2.0g, 5.2 mmol), di-p-tolylamine (1.2g, 6.1 mmol, 1 .2 eq.), tris(dibenzylideneacetone)dipalladium (0) (0.07g, 0.08 mmol, 3%Pd), sodium-t-butoxide (0.8g, 8.3 mmol, 1.6 eq.) were suspended in anhydrous toluene (65 ml). Then, a tri-t-butylphosphine/toluene solution (66 wt%, 0.04 ml, 0.13 mmol, 0.8 eq. to Pd) was added, followed by reflux for 11 hours. The reaction mixture was filtered out through a silica gel pad, and washed with toluene (150ml). The solvent was distilled off from the filtrate, and the resulting dark red oil was purified by column chromatography (silica gel/hexane + 17% dichloromethane), whereby a red solid (2.4g, 93%) was obtained.

$^1$H-NMR(400 MHz, $CDCl_3$, TMS) δ: 2.22(6H,s), 6.97(4H,d,J=8Hz), 7.01 (4H,d,J=8Hz), 7.45(1 H,t,J=7Hz), 7.50-7,54(3H,m), 7.68(1H,t,J=7Hz), 7.94(2H,r,J=8Hz), 7.98(1H,d,J=6Hz), 8.02(1H,d,J=7Hz), 8.31(1H,d,J=9Hz), 8.37 (1H,s),8,76(1H,s), 8.96(1H,d,J=9Hz)

- FDMS: calculated value $C_{38}H_{27}N$=497, measured value m/z=497 ($M^+$, 100)
- HPLC: 99.2% (detected wavelength 254 nm: area%)
- Absorption maximum wavelength: 514 nm (dichloromethane)
- Fluorescence maximum wavelength: 621 nm (dichloromethane)

The resulting solid (0.67g) was purified by sublimation under conditions of 300°C/5.3 × $10^{-3}$ Pa, whereby a red solid (0.64g) was obtained.

- HPLC:98.0% (detection wavelength 254 nm: area %)

It was revealed that, as compared with the conventional polyacene compound or the perylene-based compound, the compound or material of the invention is easy to be purified or handled, and hence, it can be synthesized at a purity of 98% or more.

[Fabrication of an organic thin film solar cell]

Examples 3 to 5

A glass substrate of 25 mm by 75 mm by 0.7 mm thick with an ITO transparent electrode was subjected to ultrasonic cleaning with isopropyl alcohol for 5 minutes, and cleaned with ultraviolet rays and ozone for 30 minutes. The substrate with transparent electrode lines thus cleaned was mounted on a substrate holder in a vacuum deposition apparatus. A p-layer compound shown in Table 1 (compound A or B synthesized in Examples 1 and 2) was formed by the resistance heating deposition at a deposition rate of 1 Å/s to form a p layer having a thickness of 30 nm so as to cover the surface of the substrate on which the transparent electrode lines were formed as a lower electrode. Subsequently, on this the n-layer compound shown in Table 1 (C60 or C70 shown below) was formed into a 60 nm-thick film by the resistance heating deposition at a deposition rate of 1 Å/s. Then, as the buffer layer, a 10 nm-film of bathocuproin (BCP) was formed at a deposition 1 Å/s. Finally, metal Al was deposited on the buffer layer as the opposing electrode

having a film thickness of 80 nm, whereby an organic thin film solar cell having an area of 0.5 cm$^2$ was formed.

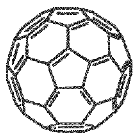
C60

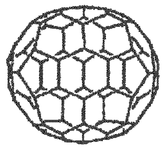
C70

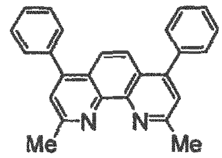
BCP

I-V characteristics were determined for the organic solar cell thus fabricated under a condition of AM 1.5 (light intensity (Pin): 100 mW/cm$^2$). From the results, the open-circuit voltage (Voc), the short-circuit current density (Jsc) and the fill factor (FF value) were obtained, and the photoelectric conversion efficiency (η) was calculated by the following equation: The results are shown in Table 1.

$$\eta\ (\%) = \frac{Voc \times Jsc \times FF}{Pin} \times 100$$

wherein Voc is the open-circuit voltage, Jsc is the short-circuit current density, FF is the fill factor and Pin is the incident light energy.
From the above equation, it can be understood that a compound having a large Voc, a large Jsc and a large FF for the same Pin has an excellent conversion efficiency.

Comparative Example 1

An organic solar cell was fabricated in the same manner as in Example 3, except that mTPD was used as the p-layer compound. The results are shown in Table **1**.

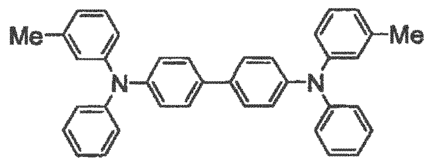
mTPD

Comparative Example 2

An organic solar cell was fabricated in the same manner as in Comparative example 1, except that the n-layer compound was changed from C60 to C70. The results are shown in Table 1.
Table 1

| | p-layer compound | n-layer compound | Voc (V) | Jsc (mA/cm$^2$) | FF | η (%) |
|---|---|---|---|---|---|---|
| Example 3 | Compound A | C60 | 1.00 | 4.25 | 0.57 | 2.43 |
| Example 4 | Compound A | C70 | 0.99 | 7.35 | 0.58 | 4.20 |
| Example 5 | Compound B | C60 | 0.93 | 3.31 | 0.53 | 1.64 |
| Com. Ex.1 | mTPD | C60 | 0.71 | 0.71 | 0.34 | 0.17 |
| Com. Ex. 2 | mTPD | C70 | 0.85 | 4.39 | 0.29 | 1.07 |

As is understood from the results shown in Table 1, the compound of the invention has improved conversion efficiency as compared with conventional amine compounds (the compounds in Comparative Examples), and hence, it

shows excellent solar cell properties.

Industrial Applicability

The dibenzofluoranthene compound of the invention can be used in an organic electronics material and an organic semiconductor material. For example, it can be used in an organic electroluminescence material, an organic field effect transistor material, an organic solar cell material, or the like. In particular, when used as a material for an organic thin film solar cell material, it shows highly efficient energy conversion characteristics.
The dibenzofluoranthene compound of the invention is particularly useful as the material for the p layer of an organic thin film solar cell.
The organic thin film solar cell of the invention can be used in a clock, a mobile cell, a mobile personal computer, or the like.
Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.
The documents described in the specification are incorporated herein by reference in its entirety.

## Claims

1. A dibenzofluoranthene compound represented by the following formula (A):

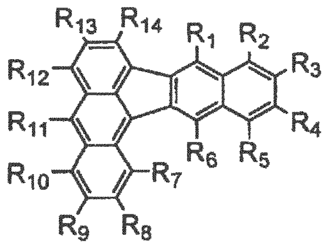

(A)

wherein at least one of $R_1$ to $R_{14}$ is an amino group represented by the following formula (S);
$R_1$ to $R_{14}$ that is not an amino group represented by the formula (S) are independently a hydrogen atom, a $C_1$-$C_{40}$ substituted or unsubstituted alkyl groups, a $C_2$-$C_{40}$ substituted or unsubstituted alkenyl group, a $C_2$-$C_{40}$ substituted or unsubstituted alkynyl group, a $C_6$-$C_{40}$ substituted or unsubstituted aryl groups, a $C_3$-$C_{40}$ substituted or unsubstituted heteroaryl group, a $C_1$-$C_{40}$ substituted or unsubstituted alkoxy group on a $C_6$-$C_{40}$ substituted or unsubstituted aryloxy group;

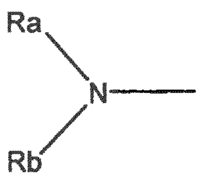

(S)

wherein in $R_a$ and $R_b$ are independently a $C_6$-$C_{40}$ substituted or unsubstituted group or a $C_1$-$C_{40}$ substituted or unsubstituted group; and $R_a$ and $R_b$ may combine with each other to form a ring.

2. The dibenzofluoranthene compound according to claim 1 which is represented by the following formula (B):

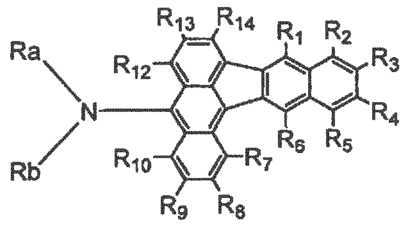

(B)

wherein $R_1$ to $R_{10}$ and $R_{10}$ to $R_{14}$ are independently a hydrogen atom, a $C_1$-$C_{40}$ substituted or unsubstituted alkyl group, a $C_2$-$C_{40}$ substituted or unsubstituted alkenyl group, a $C_2$-$C_{40}$ a substituted or unsubstituted alkynyl group, a $C_6$-$C_{40}$ substituted or unsubstituted aryl group, a $C_3$-$C_{40}$ substituted or unsubstituted heteroaryl group, a $C_1$-$C_{40}$ substituted or unsubstituted alkoxy group or a $C_6$-$C_{40}$ substituted or unsubstituted aryloxy and
$R_a$ and $R_b$ are a $C_6$-$C_{40}$ substituted or unsubstituted aryl group or a $C_1$-$C_{40}$ substituted or unsubstituted alkyl group; and $R_a$ and $R_b$ may combine with each other to form a ring.

**3.** The dibenzofluoranthene compound according to claim 1 or 2 which is represented by the following formula (C):

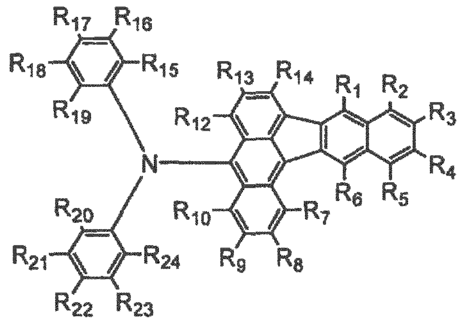

(C)

wherein $R_1$ to $R_{10}$ and $R_{12}$ to $R_{24}$ are independently a hydrogen atom, a $C_1$-$C_{40}$ substituted or unsubstituted alkyl group, a $C_6$-$C_{40}$ substituted or unsubstituted aryl group, a $C_3$-$C_{40}$ substituted or unsubstituted heteroaryl group or a $C_1$-$C_{40}$ substituted or unsubstituted alkoxy group,

**4.** An organic thin film solar cell material comprising the dibenzofluororanthene compound according to any of claims 1 to 3.

**5.** An organic thin film solar cell comprising the dibenzofluororanthene compound according to any of claims 1 to 3.

**6.** The organic thin film solar cell according to claim 5 which comprises a p layer, and the p layer being a layer which comprises the dibenzofluoranthene compound.

**7.** The organic thin film solar cell according to claim 6 which further comprises an n layer, and the n layer being a layer which comprises a fullerene derivative.

## INTERNATIONAL SEARCH REPORT

International application No.
PCT/JP2011/000408

A. CLASSIFICATION OF SUBJECT MATTER
*C07C211/61*(2006.01)i, *H01L29/786*(2006.01)i, *H01L51/05*(2006.01)i, *H01L51/30*(2006.01)i, *H01L51/42*(2006.01)i

According to International Patent Classification (IPC) or to both national classification and IPC

B. FIELDS SEARCHED

Minimum documentation searched (classification system followed by classification symbols)
C07C211/61, H01L29/786, H01L51/05, H01L51/30, H01L51/42

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2005-320286 A (Chemipro Kasei Kaisha, Ltd.), 17 November 2005 (17.11.2005), claim 1; application example 26 (Family: none) | 1-7 |
| A | JP 11-054282 A (Mitsui Chemicals, Inc.), 26 February 1999 (26.02.1999), claims 1, 6; examples 1 to 9 (Family: none) | 1-7 |
| A | JP 2009-266955 A (Idemitsu Kosan Co., Ltd.), 12 November 2009 (12.11.2009), claims 1, 8; synthesis examples 4, 5 & WO 2009/130991 A1 | 1-7 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
12 April, 2011 (12.04.11)

Date of mailing of the international search report
26 April, 2011 (26.04.11)

Name and mailing address of the ISA/
Japanese Patent Office

Facsimile No.

Authorized officer

Telephone No.

Form PCT/ISA/210 (second sheet) (July 2009)

INTERNATIONAL SEARCH REPORT

International application No.
PCT/JP2011/000408

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-290091 A (Idemitsu Kosan Co., Ltd.), 10 December 2009 (10.12.2009), claims 1, 5; preparation examples 1, 2; examples 1, 2 (Family: none) | 1-7 |
| A | JP 2008-263112 A (Canon Inc.), 30 October 2008 (30.10.2008), claim 1; paragraphs [0032] to [0035] (Family: none) | 1-7 |
| A | JP 2009-188136 A (Idemitsu Kosan Co., Ltd.), 20 August 2009 (20.08.2009), claims 1, 9; examples 5 to 7 (Family: none) | 1-7 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008091380 A
- JP 2008135540 A